Europäisches Patentamt

European Patent Office

Office européen des brevets

(11) Publication number: **0 489 977 A1**

# EUROPEAN PATENT APPLICATION

(21) Application number: **90203253.1**

(51) Int. Cl.⁵: **A61B 5/14**

(22) Date of filing: **11.12.90**

(43) Date of publication of application:
**17.06.92 Bulletin 92/25**

(84) Designated Contracting States:
**AT BE CH DE DK ES FR GB GR LI LU NL SE**

(71) Applicant: **Leopardi, Francesco**
**Via Mosè Bianchi, 49**
**I-20149 Milano(IT)**
Applicant: **Paoletti, Sergio**
**Via Palatino, 3**
**I-20148 Milano(IT)**

(72) Inventor: **Leopardi, Francesco**
**Via Mosè Bianchi, 49**
**I-20149 Milano(IT)**
Inventor: **Paoletti, Sergio**
**Via Palatino, 3**
**I-20148 Milano(IT)**

(74) Representative: **Marchi, Massimo et al**
**c/o Marchi & Mittler s.r.l. Viale Lombardia 20**
**I-20131 Milano(IT)**

(54) **Device for taking blood from a patient and for transferring it to a test tube under vacuum.**

(57) The hollow tubular device 1 has one extremity 2 open and communicating with the outside and one closed by a flat end wall 3 which is substantially perpendicular to the longitudinal axis of the device 1 and is traversed by a hole 4; from the external surface of said end wall 3 there extends, towards the outside and all round said hole 4, a conical coupling 5 for an injection needle of a standard type and, in the proximity of said end wall 3, the internal tubular wall of said device 1 has a circumferential relief 6 which defines a seat suitable for sealingly receiving the base 7 of a hollow needle 8, said base 7 having a surface substantially equal to that of said end wall 3 against which it goes to coincide.

Fig.1

The present invention relates to a device for taking a certain quantity of blood from a vein of a patient and for transferring it to a test tube closed by an elastic and pierceable plug in which a vacuum has previously been created to a predetermined value such that an accurate quantity of blood is sucked into said test tube.

It is known that the primary requirement of anybody operating nowadays in the field of clinical and medical analyses is that of avoiding even the smallest contact with the blood to be analysed so as to prevent any contagion.

It is also known that for taking blood samples, instead of the traditional disposable syringes, the use is becoming increasingly common of test tubes sealed by elastic pierceable plugs in which a vacuum has previously been created to a predetermined value such that an accurate quantity of blood, generally 2.5, 5 or 10 ml, is sucked into said test tube.

At the moment when blood is taken from the patient, the use is provided for of a hollow tubular device called a holder, generally of a rigid and transparent polymer material, having a tubular shape, with one extremity open and communicating with the outside and one closed by a flat end wall which is substantially perpendicular to the axis of the device and is traversed by a threaded hole in which there is screwed a centrally threaded casing integral with a metal needle with two tips, commonly known as a disposable twin-tip needle. After the central threaded casing of the twin-tip needle has been screwed into the threaded hole of said holder, one half of the twin-tip needle extends axially outside while the other half of said needle extends axially in the tubular cavity of said holder. The tip of the external half of said disposable twin-tip needle is introduced into a vein of the patient while the tip of the half internal to said holder is introduced into the pierceable plug of a test tube under vacuum by pushing the upper part of said test tube completely into the tubular cavity of said holder, whose internal diameter is only slightly larger than the external diameter of said test tube, while its length is less than that of the test tube. At this point, the vacuum having a predetermined value in the test tube sucks the predetermined quantity of blood into the test tube itself.

Once the operation is over, the test tube containing the desired quantity of blood is sent to the analysis laboratory as it is, already plugged, and the twin-tip needle is thrown away.

This known holder device, however, also has some drawbacks. A first drawback is constituted by the cost of the disposable metal twin-tip needle. A further drawback is represented by the fact that the disposable metal twin-tip needle is necessarily mounted at the centre of the end wall so as to be able to pierce said plug in a position very close to the centre of the plug itself but this central position of the needle with respect to the holder, causes the angle of entry into the vein of the external section of said needle to be fairly wide and this makes the introduction more complex and dangerous than it might be if the needle were arranged in a peripheral position with respect to said holder.

The present invention thus propose to overcome the above drawbacks by means of a hollow tubular device to take a certain quantity of blood from a vein of a patient and to transfer it to a test tube, which has an external diameter only slightly smaller than the internal diameter of the tubular cavity of said device and is closed by an elastic and pierceable plug, in which a vacuum has previously been created to a predetermined value such that an exact quantity of blood is sucked into said test tube, said device having one extremity open and communicating with the outside for the introduction of the upper part of said test tube into the tubular cavity of said device and one extremity closed by a flat end wall which is substantially perpendicular to the longitudinal axis of the device and is traversed by a hole, characterised in that from the external surface of said end wall there extends, towards the outside and all round said hole, a conical coupling for an injection needle of a standard type for the introduction into a vein of a patient and which, in the proximity of said end wall, the internal tubular wall of said device has a circumferential relief which defines a seat suitable for sealingly receiving the base of a hollow needle capable of perforating said plug when the upper part of said test tube is pushed completely into the tubular cavity of said device said base having a surface substantially equal to that of said end wall against which it goes to coincide.

The present invention thus provides for the use of two different needles rather than one twin-tip needle. The external needle according to the present invention is a steel needle of a standard type for injections; the internal needle is also made of steel or, even more preferably, of a rigid polymer material and is characterised by a wide base having substantially the same shape as the internal surface of said end wall against which it sealingly coincides. The cost of these two needles is considerably lower than the cost of a twin-tip needle with a threaded central casing.

Preferably the base of the internal needle has a seat for receiving an elastic seal which ensures the seal between the base of the internal needle and said end wall.

According to the present invention the two needles need not necessarily be aligned along the longitudinal axis as is the case, on the other hand, with devices of the known type. Thus, the hole through said end wall can traverse it either at the centre or in a peripheral position. As a matter of fact, the more said hole - and thus the conical coupling for the external needle - is in a peripheral position, the smaller is the angle of introduction into the vein of the external needle mounted on said conical coupling. Thus, the arrangement of the conical coupling in a peripheral position is provided for as a preferred embodiment and constitutes another of the advantages of the device of the present invention.

The internal needle, on the other hand, is preferably still arranged along the longitudinal axis of the tubular cavity so as to penetrate into the pierceable plug of the test tube under vacuum in a position very close to the centre of the plug as in the case of the twin-tip needle. When the hole in the end wall and thus the conical coupling for the external needle are arranged peripherally, the communication between the cavity of the internal needle in an axial position and that of the external needle mounted on the conical coupling in a peripheral position is obtained by meansof a small groove, obtained on the surface of the base of the internal needle, such as to form, when said base is in contact with said end wall, a channel which allows the cavities of the two needles to communicate with each other.

These and other features and advantages of the device according to the present invention shall appear even more evident from the following description of an embodiment according to the present invention illustrated in the enclosed table of drawings, wherein:

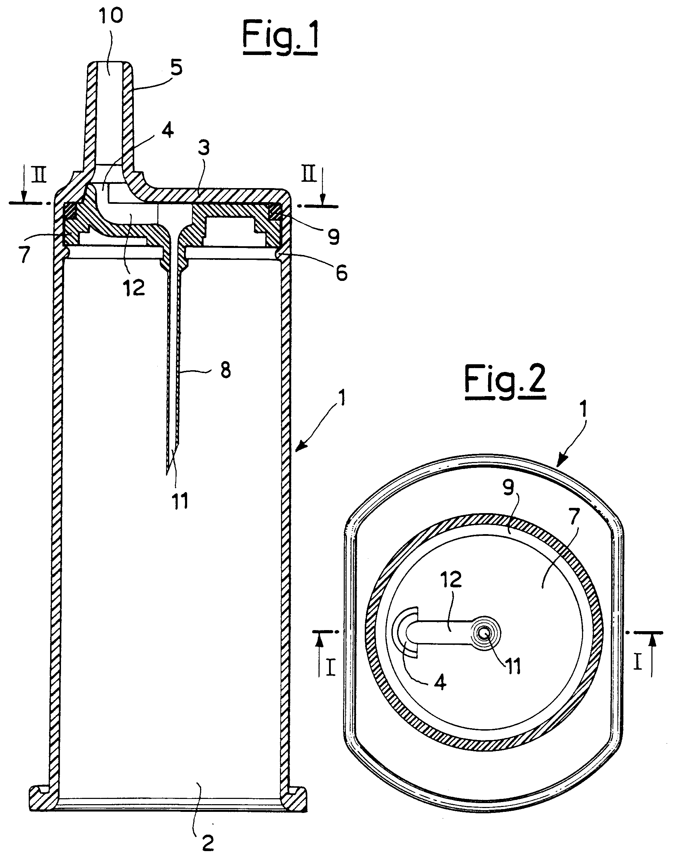

Fig. 1 is a longitudinal sectional view, taken along the line I-I of Fig. 2, of a device according to the present invention;

Fig. 2 is a transverse sectional view taken along the line II-II of Fig.1.

As can be seen from Fig.s 1 and 2, the device 1 illustrated in the enclosed table is constituted by a hollow tubular casing which has the extremity 2 open and communicating with the outside while the other extremity is closed by an end wall 3, flat which is substantially perpendicular to the longitudinal axis of said hollow tubular casing and is traversed by a hole 4.

From the external surface of said end wall 3 there extends, towards the outside and all round said hole 4, a conical coupling 5 for an injection needle of a standard type. In addition, in the proximity of said end wall 3, the internal tubular wall of said device 1, has a circumferential relief 6 which defines a seat suitable for sealingly receiving the base 7 of a hollow needle 8. Said base 7 has a surface substantially equal to that of the end wall 3 against which it goes to coincide.

In addition, along the circumference of said base 7 there is a seat which receives an elastic seal 9 to ensure the seal between the base 7 of the internal needle 8 and the end wall 3.

In the embodiment illustrated in Fig.s 1 and 2, the conical coupling 5 has not been obtained at the centre of the end wall 3, thus the cavity 10 of the conical coupling 5 is not aligned with the cavity 11 of the needle 8. In the base 7 of the internal needle 8 there is a small groove 12 which, in contact with the end wall 3, forms a channel for the passage of the blood from the cavity 10 of said external conical coupling 5 to the cavity 11 of said needle 8 housed in said device 1.

This peripheral arrangement of the conical coupling 5 has the advantage that the injection needle of a standard type (not illustrated) mounted on it can be introduced into the vein with an entry angle which is less than when the conical coupling 5 is positioned at the centre of the end wall 3.

At the moment of use, the injection needle of a standard type (not illustrated) is mounted on the conical coupling 5 and said external needle is introduced into a vein of a patient; at the same time there is introduced into the open extremity 2 of the device 1 the upper part of a test tube under vacuum closed by a pierceable plug (not illustrated) and it is pushed against the needle 8 until this has perforated said plug and it has penetrated into the test tube. At this point the vacuum previously created in the test tube sucks in the pre-set quantity of blood along the following path: vein, cavity of the external needle (not illustrated), cavity 10 of the conical coupling 5, channel formed by the groove 12 of the base 7 against the end wall 3, cavity 11 of the internal needle 8, test tube (not illustrated). After this operation is over, the test tube, containing the exact pre-set quantity of blood is removed from the device thus also causing the withdrawal of the internal needle 8 from the plug which remains integral with the test tube and the latter is sent to the analysis laboratory as it is, already plugged.

For the sake of convenience, Fig.s 1 and 2 show an enlargment of an embodiment of the device according to the present invention.

Examples of the typical dimensions of a device according to the present invention (to be used with test tubes under vacuum with a length of 10 cm and an external diameter of 1.6 cm) are: length 6 cm, internal diameter 1.7-1.8 cm.

**Claims**

1. Hollow tubular device 1 to take a certain quantity of blood from a vein of a patient and to transfer it to a test tube, which has the external diameter only slightly smaller than the internal diameter of the tubular cavity of said device and is closed by an elastic and pierceable plug, in which a vacuum has previously been created to a predetermined value such that an exact quantity of blood is sucked into said test tube, said device 1 having one extremity 2 open and communicating with the outside for the introduction of the upper part of said test tube into the tubular cavity of said device and one extremity closed by a flat end wall 3 which is substantially perpendicular to the longitudinal axis of the device 1 and is traversed by a hole 4, characterised in that from the external surface of said end wall 3 there extends, towards the outside and all round said hole 4, a conical coupling 5 for an injection needle of a standard type for the introduction into a vein of a patient and which, in the proximity of said end wall 3, the internal tubular wall of said device 1 has a circumferential relief 6 which defines a seat suitable for sealingly receiving the base 7 of a hollow needle 8 capable of perforating said plug when the upper part of said test tube is pushed completely into the tubular cavity of said device, said base 7 having a surface substantially equal to that of said end wall 3 against which it goes to coincide.

2. Device according to the previous claim 1, characterised in that along the circumference of said base of the needle 8 housed inside said device 1 there is a seat which receives an elastic seal 9 to ensure the seal between said base 7 and the end wall 3 of said device 1.

3. Device according to the previous claim 2, characterised in that when the cavity 10 of said conical coupling 5 and the cavity 11 of the needle 8 housed inside said device 1 are not aligned one with the other, there is a small groove 12 in said base 7 to form, when said base 7 is in contact with said end wall 3, a channel for the passage of the blood from the cavity 10 of said external conical coupling 5 to the cavity 11 of said needle 8 housed in said device 1.

Fig.1

Fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-70356 (BECTON,DICKINSON AND COMPANY) * page 10, lines 6 - 14 * | 1 | A61B5/14 |
| A | * page 11, lines 20 - 23; figures 2, 4 * | 2 | |
| Y | EP-A-224604 (INTERMEDICAT GMBH) * figure 1 * | 1 | |
| A | | 3 | |
| A | WO-A-8910723 (NIELSEN) * page 6, line 32 - page 8, line 15; figures * | 1, 3 | |

TECHNICAL FIELDS
SEARCHED (Int. Cl.5 )

A61B

The present search report has been drawn up for all claims

| Place of search | Date of completion of the search | Examiner |
|---|---|---|
| THE HAGUE | 02 AUGUST 1991 | PINEAU A. |

EPO FORM 1503 03.82 (P0401)